# EUROPÄISCHE PATENTANMELDUNG

(11) **EP 0 847 776 A1**
(43) Veröffentlichungstag der Anmeldung: **17.06.1998**
(21) Anmeldenummer: 97121873.0
(22) Anmeldetag: 11.12.1997
(51) Int. Cl.: A61N 1/36, A61B 5/0456

(54) **Vorrichtung zum Stimulieren eines Muskels oder von Muskelgruppen**

(30) Priorität: 11.12.1996 DE 19651600
(71) Anmelder: Bavaria Patente und Lizenzen Verwertungsgesellschaft mbH, 01731 Kreischa (DE)
(72) Erfinder: Blümel, Georg, Doz.Dr..sc.nat., 04209 Leipzig (DE)
(74) Vertreter: von Samson-Himmelstjerna, Friedrich R., Dipl.-Phys.

(57) **Zusammenfassung**

Die Erfindung betrifft eine Vorrichtung (1) zum Stimulieren wenigstens eines Muskels oder einer Muskelgruppe für diagnostische und/oder therapeutische Zwecke, insbesondere zur Förderung der Durchblutung in dem stimulierten Muskelbereich (26, 27), welche periodisch elektrische Impulse - (wenigstens) ein Stimulationssignal (9) - von einem Impulsgenerator (3, 4) über extern auf die Haut des Benutzers an dem zu stimulierenden Bereich (26, 27) plazierten Elektroden (18-23) auf den zu stimulierenden Muskel bzw. die zu stimulierende Muskelgruppe überträgt, wobei die Parameter des Impulsgenerators (3, 4), wie Impulsfrequenz, Impulsamplitude, Impulsspannung, Impulsdauer u.a., von mindestens einem Steuergerät (10) aus steuerbar sind, mit einer derartigen Auslegung des Steuergerätes (10), daß es zur Steuerung zumindest der Impulsfrequenz ein Steuersignal aus einem bestimmten Bereich periodisch auftretender elektrischer Signalzyklen der Herzaktion(en) - dem Herzsignal (6) -, insbesondere der R-Zacke des Elektrokardiogramms, eines Benutzers (5) ableitet, derart, daß zwischen dem Herzsignal (6) und dem Stimulationssignal (9) eine zeitliche Abhängigkeit, insbesondere eine weitgehende Übereinstimmung (Synchronisation), herrscht.

## Beschreibung

Die Erfindung betrifft eine Vorrichtung zum Stimulieren von mindestens einem Muskel für diagnostische und/oder therapeutische Zwecke mit den im Oberbegriff des Anspruchs 1 angegebenen Merkmalen.

Bei Vorrichtungen dieser Art, wie sie z.B. durch die US-PS 5 358 513 (POWELL) oder die AT-PS 47 796 (KLOTZ) oder die PCT-Anmeldung WO 90/07955 (GRENFELL) bekanntgeworden sind, wird die Steuerung der verschiedenen Parameter je nach dem diagnostischen oder therapeutischen Effekt so vorgenommen, daß während der Behandlung über eine Tastatur oder vorherige Eingabe in einen Progammspeicher Erfahrungswerte in den Impulsgenerator eingespeist werden, wobei sich diese Werte in weiten Grenzen halten; z.B. liegt die Impulsfrequenz bzw. -periode nach der erstgenannten Druckschrift (POWELL) etwa bei 2500 Hertz, nach der zweitgenannten Veröffentlichung (KLOTZ) zwischen 125 ms und 2125 ms und nach der drittgenannten (GRENFELL) Druckschrift zwischen 2 und 20 Hertz. In allen Fällen findet die Tatsache keine Beachtung, daß das Herz der stimulierten Person seinerseits als eine Art Impulsgenerator angesehen werden könnte, dessen Impulse Muskelkontraktionen, nämlich die des Herzmuskels, hervorrufen und so indirekt über die Blutdruckwelle auch auf die stimulierten Muskelpartien einwirken.

Bei den bekannten Vorrichtungen ist es höchst unwahrscheinlich, daß der durch die externe Muskelstimulation verbesserte Blutdurchsatz durch den Muskel zusätzlich noch durch die vom Herzen stammende Blutdruckwelle verstärkt wird. Wie bereits erwähnt, sind nämlich die zusammenwirkenden Frequenzen zu verschieden, so daß eher Interferenz- als Resonanzerscheinungen auftreten werden.

Der Erfindung zielt darauf ab, die eingangs genannte gattungsgemäße Vorrichtungen weiterzubilden.

Sie erreicht dieses Ziel durch den Gegenstand des Anspruchs 1, also durch eine Vorrichtung zum Stimulieren wenigstens eines Muskels oder einer Muskelgruppe für diagnostische und/oder therapeutische Zwecke, insbesondere zur Förderung der Durchblutung im stimulierten Muskelbereich, welche periodisch elektrische Impulse - (wenigstens) ein Stimulationssignal - von einem Impulsgenerator über extern auf die Haut des Benutzers an dem zu stimulierenden Bereich plazierten Elektroden auf den zu stimulierenden Muskel bzw. die zu stimulierende Muskelgruppe überträgt, wobei die Parameter des Impulsgenerators, wie Impulsfrequenz, Impulsamplitude, Impulsspannung, Impulsdauer u.a., von mindestens einem Steuergerät aus steuerbar sind. Dabei ist das Steuergerät derart ausgelegt, daß es zur Steuerung zumindest der Impulsfrequenz ein Steuersignal aus einem bestimmten Bereich periodisch auftretender elektrischer Signalzyklen der Herzaktion(en) - dem Herzsignal -, insbesondere der R-Zacke des Elektrokardiogramms, eines Benutzers ableitet, derart, daß zwischen dem Herzsignal und dem Stimulationssignal eine zeitliche Abhängigkeit, insbesondere eine weitgehende Übereinstimmung (Synchronisation), herrscht.

Die erfindungsgemäße Vorrichtung beaufschlagt in Abhängigkeit der Herztätigkeit eines Benutzers (bspw. eines Patienten) einzelne oder mehrere Muskeln oder Muskelgruppen jeweils interessierender Körperpartien mit elektrischen Impulsen. Diese Beaufschlagung stellt eine Stimulation der betroffenen Muskeln dar. Als Folge der Stimulation kontrahieren die Muskeln, was zu einer erhöhten Durchblutung in den stimulierten Bereichen führt.

Zusätzlich üben die Muskeln durch die Kontraktion und die nachfolgende Muskelentspannung einen Pumpeffekt auf die Blut- und Lymphgefäße aus. Da beide Gefäßsysteme mit "Einwegventilen" (Gefäßklappen) versehen sind, wird hierdurch der Kreislauf der jeweiligen Flüssigkeit (Blut bzw. Lymphe) nur in der gewünschten Richtung gefördert. Dabei erreicht man eine Unterstützung des Herz-Kreislaufsystems und des Lymphkreislaufs.

Außerdem hat die evocierte Muskelkontraktion einen Trainingseffekt auf die Muskulatur selbst. Sie beugt daher einer etwaigen Muskeldegeneration vor. Ferner kann die erfindungsgemäße Vorrichtung bei bettlägrigen Patienten einem Wundliegen (sog. Decubitus-Effekt) vermeiden helfen, indem sie gefährdete Körperpartien durch gezielte Muskelstimulation beansprucht und trainiert.

Als besonderer Vorteil sind bei der Erfindung die elektrischen Stimulationsimpulse zeitlich auf den Herzrhythmus abgestimmt. Vorzugsweise entspricht die Impulsfrequenz der Herzfrequenz. Dabei erfolgt eine permanente Anpassung der Impulsfrequenz an die Herzfrequenz, d.h. ändert sich die Herzfrequenz, wird auch die Impulsfrequenz verändert. Die Impulsfrequenz unterliegt also einer Regelung.

Die Impulsfrequenz kann aber auch so gewählt werden, daß die Herzfrequenz ein ganzzahliges Vielfaches der Impulsfrequenz beträgt. Dann erfolgt eine Stimulation z.B. nur bei oder nach jedem zweiten (oder dritten, vierten, ...) Herzschlag. Dadurch erreicht man eine Reduzierung der Simulationsimpulse und kann ein evtl. Überbeanspruchung der betroffenen Muskelpartien vermeiden.

Die Erfindung erlaubt ferner die externen Stimulationsimpulse so zu steuern, daß eine Interferenz der beiden Impulssysteme - Herz und Impulsgenerator - mit Sicherheit vermieden wird und damit die Durchblutung der betreffenden Muskelbereiche optimiert wird. Zugleich vermeidet die Erfindung, daß die Stimulationsimpulse sich störend auf die Herzfunktion im Sinne einer Herzrhythmusstörung auswirken können.

In der DD-PS 73 846 (SCHUMANN) ist zwar angegeben, daß es bekannt sei, die periodisch mit jeder Herzaktion auftretende R-Zacke aus einem Elektrokardiogramm zur Triggerung für diagnostische oder therapeutische Geräte zu verwenden. Jedoch ist dort nur ein einziges derartiges Gerät genannt, nämlich ein Kardiotachometer, mit dem lediglich die jeweilige Herzfrequenz pro Zeiteinheit angezeigt werden soll.

Auch aus der US-PS 43 31 157 (KELLER) geht kein Hinweis in Richtung auf die erfindungsgemäße Lösung hervor, weil nach dieser Druckschrift vermieden werden soll, daß bei postoparativer Behandlung eines Patienten mit Muskelstimulation (zur Schmerzmilderung) die auf einem Oszillographen oder dergleichen wiedergegebenen Stimulationskurven mit den ebenfalls auf dem Oszillographen wiedergegebenen Elektrokardiogrammkurven Interferenzen bilden und dadurch ein ordnungsgemäßes Ablesen in Frage stellen.

Bei einer bevorzugten Ausgestaltung der Erfindung berücksichtigt die zeitliche Abhängigkeit die Laufzeit einer Blutdruckwelle vom Herzen zum/zur zu stimulierenden Muskel/Muskelgruppe, so daß die Impulse entsprechend zeitlich versetzt ausgelöst werden (Anspruch 2). Da sich eine Blutdruckwelle im Herz-Kreislaufsystem mit einer endlichen (relativ langsamen) Geschwindigkeit ausbreitet, treten nennenswerte Verzögerungen - sog. Laufzeiten - auf, bis eine Blutdruckwelle einen bestimmten Körperbereich erreicht. Die Laufzeit ist um so größer, je entfernter der jeweilige Körperbereich vom Herz ist. Um einen optimierten (bzw. optimalen) Pumpeffekt zu erreichen, ist die Stimulation also nicht nur auf den Herzschlag, sondern auch auf die Laufzeit abgestimmt. Ist die Abstimmung optimal, erreicht man eine maximale Unterstützung der Durchblutung auch in vom Herz entfernt gelegenen Körperbereichen.

Außerdem kann man durch die Abstimmung eine unerwünschte, kontraproduktive Anregung des Bluttransports verhindern, also eine Anregung die dem natürlichen Bluttransport entgegenwirkt. Eine solche kontraproduktive Anregung kann z.B. dann entstehen, wenn eine Muskelkontraktion eine Blutdruckwelle abschnürt. Dadurch wird der natürliche Bluttransport vermindert oder im schlimmsten Fall unterbrochen. Die Abstimmung verhindert diesen unerwünschten Effekt.

Besonders bevorzugt werden bei der Stimulation von zwei oder mehr Muskeln/Muskelgruppen diese mit mehreren, synchronen oder zeitlich versetzten Stimulationssignalen, insbesondere mittels eines Multiplexers, stimuliert (Anspruch 3). Vorteilhafterweise erfolgt die Stimulation mehrerer Muskelpartien an verschiedenen Stellen des Körpers. Haben dabei die zu stimulierenden Muskelpartien unterschiedliche Entfernungen vom Herz, erfolgt die Stimulation mit mehreren zeitlich versetzten Stimulationssignalen. Bei Muskelpartien mit gleichen Entfernungen von Herz kann die Stimulation mit demselben Stimulationssignal erfolgen.

Bevorzugt werden die zeitlich versetzten Stimulationssignale mit einem Multiplexer erzeugt. Dieser Multiplexer tastet zeitlich nacheinander z.B. ein Gleichspannungssignal ab, so daß eine Folge zeitlich versetzter Impulse entsteht. Die Vorrichtung leitet dann je einen Impuls an je eine zu stimulierende Muskelpartie.

Vorteilhaft werden die Muskeln/Muskelgruppen derart zeitlich versetzt stimuliert, daß Blut und/oder Lymphe in eine bestimmte, insbesondere dem natürlichen Kreislauf entsprechende, Richtung transportiert bzw. gepumpt wird (Anspruch 4). Eine aufeinanderfolgende Stimulation benachbarter Muskelpartien erzeugt eine Welle in den Blut- oder Lymphgefäßen entlang den stimulierten Muskelpartien. Die Richtung ist grundsätzlich unabhängig von der Richtung des natürlichen Kreislaufes von Blut oder Lypmhe. Jedoch ist bei einem gesunden Herz-Kreislaufsystem die Richtung durch die als Einwegventile wirkenden Gefäßklappen vorgegeben. Krankhafte Gefäßklappen sind allerdings nicht immer in der Lage vollständig zu schließen, so daß sie ihre Einwegfunktion nicht mehr erfüllen können. Blut oder Lymphe fließen dann auch entgegen der natürlichen Richtung. Dies kann zu einem Stau (infolge der Erdgravitation) in tiefergelegenen Körperpartien führen, beispielsweise in den Beinen. In diesem Fall ist es besonders vorteilhaft, die Blut- bzw. Lymphzirkulation derart anzuregen, daß Blut bzw. Lymphe nicht wieder zurückfließt, sondern in die dem natürlichen Kreislauf entsprechende Richtung gedrückt wird. Man erreicht dies durch die zeitlich aufeinanderfolgende Stimulation von parallel zu den betroffenen Blut- oder Lymphgefäßen verlaufenden Muskeln.

Bevorzugt sind mehrere Elektrodenpaare vorgesehen (Anspruch 5). Um die Stimulation verschiedener Muskelpartien zu gewährleisten ist, weist diese Ausgestaltung mehrere Elektrodenpaare auf. Dabei erfolgt die Stimulation jeder Muskelpartie mit jeweils wenigstens einem Elektrodenpaar. Jedes Elektrodenpaar erlaubt das Aufbringen, insbesondere verschiedener, elektrischer Impulse auf die Haut einer entsprechenden Körperpartie.

Bevorzugt weist das Steuergerät mehrere Kanäle auf, wobei an jeden Kanal ein Elektrodenpaar anschließbar ist (Anspruch 6). Jeder Kanal kann unabhängig von den anderen Kanälen elektrische Impulse erzeugen. Bevorzugt folgen jedoch die Impulse zeitlich aufeinander, so daß jeder Kanal zwar die gleiche Impulsfrequenz aber eine andere Impulsphase hat. Dabei kann jeder Kanal mit je einem - auf der Haut einer ausgewählten Körperpartie aufliegenden - Elektrodenpaar verbunden werden.

Bevorzugt umgeben die Elektroden zusammen mit wenigstens einer Manschette den zu stimulierenden Bereich (Anspruch 7). Die Elektroden sind also insbesondere in bzw. an einer oder mehreren Manschetten untergebracht. Diese Manschetten können einen Klettverschluß oder einen anderen Verschluß aufweisen. Mit derartigen Manschetten kann man vorallem Extremitäten, wie Arme und Beine umschließen, so daß die mit der Manschette verbundenen Elektroden an der jeweiligen Körperpartie nicht verrutschen, sondern fixiert sind.

Bevorzugt sind die Manschetten unelastisch ausgebildet (Anspruch 8). Eine unelastische Manschette verhindert bei einer Muskelkontraktion die Ausdehnung des Muskels nach außen, d.h. in Querrichtung. Deshalb weicht der Muskel (bei Kontraktion) in Längsrichtung aus. Dadurch erreicht man einen zusätzlicher Pumpeffekt, der den Transport von Blut bzw. Lymphe unterstützt.

Bei einer weiteren Ausgestaltung der Erfindung sind die Stimulationssignale abhängig von Gehirnströmen, beispielsweise von Schlafströmen (Anspruch 9). Die Stimulationssignale sind bei dieser Ausgestaltung also nicht allein von dem Herzsignal (insb. dem EKG) abhängig, sondern auch von den Strömen des Gehirns. Die Elektroenzephalographie (EEG) ermöglicht das Messen dieser Gehirnströme. Aus den Gehirnströmen kann man auf den Zustand des Patienten schließen, beispielsweise, ob er dazu neigt, das Bewußtsein zu verlieren oder in ein Koma zu verfallen. Daher kann man durch Heranziehen der Gehirnströme zum Bestimmen der Stimulationssignale insbesondere bei Komagefahr des Patienten einen Wachzustand aufrechterhalten.

Beim Generieren der Stimulationssignale gehen bei dieser Ausgestaltung also zwei verschiedenartige Größen, nämlich Herzsignal und Hirnströme, ein. Die Vorrichtung gemäß dieser Ausgestaltung kann diese Größen unterschiedlich gewichten, so daß die eine oder die andere Größe die Stimulationssignale mehr oder weniger stark beeinflußt. Die Gewichtungen können auch zeitlich variabel, beispielsweise bei Tag und Nacht unterschiedlich, sein. Auf diese Weise kann man unterschiedliche Hirntätigkeiten, beispielsweise während (verschiedener) Schlafphasen, berücksichtigen.

Im Rahmen der Erfindung kann (können) grundsätzlich das Stimulationssignal (die Stimulationssignale) auch allein von den Gehirnströmen abgeleitet werden, d.h., daß eine zeitliche Abhängigkeit nur zwischen Stimulationssignal(en) und Gehirnströmen besteht.

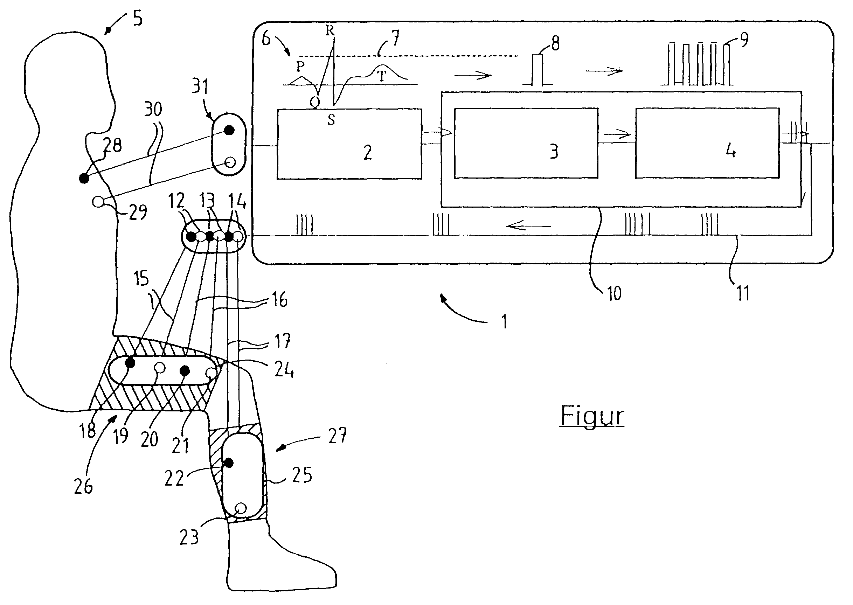

Die Erfindung wird nun anhand eines Ausführungsbeispiels und der angefügten schematischen Zeichnung näher erläutert.
- Die einzige Figur: zeigt eine schematische beispielhafte Darstellung einer Vorrichtung zum Stimulieren von Muskeln und Muskelgruppen.

Die Figur veranschaulicht eine Stimulationsvorrichtung 1, im wesentlichen bestehend aus drei Modulen, nämlich einem ersten Modul 2, einem zweiten Modul 3 und einem dritten Modul 4. Das erste Modul 2 ist ein sogenannter EKG-Verstärker. Es dient zum Erfassen der Herztätigkeit eines Benutzers oder Probanden 5 mittels eines sogenannten Elektrokardiogramms (EKG) 6. Das EKG 6 zeigt die typischen EKG-Bereiche P, Q, R, S und T. Ferner ist im EKG 6 ein Schwellwert 7 eingezeichnet. Dieser Schwellwert 7 dient zum automatischen Bestimmen der sogenannten R-Zacke des EKG 6.

Überschreitet das EKG den Schwellwert 7, wird die R-Zacke detektiert.

Das zweite Modul 3 ist der sogenannte Laufzeit- und Triggererzeuger. Dieses zweite Modul 3 erzeugt bei Überschreiten des Schwellwerts 7 einen sogenannten Triggerimpuls 8 aus der R-Zacke des EKG 6. Ferner gibt das zweite Modul 3 Laufzeiten für verschiedene Stimulationssignale vor. Basierend auf dem Triggerimpuls 8 und den Laufzeiten erzeugt das dritte Modul 4 eine Folge von Impulsen 9. Diese Folge von Impulsen 9 dient zur Stimulation von Muskeln oder Muskelgruppen. Daher wird das dritte Modul 4 auch als Elektromyostimulator bezeichnet. Es bildet die Endstufe der Stimulationsvorrichtung 1.

Das zweite Modul 3 und das dritte Modul 4 kann man auch als eine Einheit betrachten, nämlich als den sogenannten programmierbaren Elektromyostimulator 10. Er ist insofern programmierbar, als die Parameter der Impulsgenerierung, wie Impulsfrequenz, Impulsamplitude, Impulsspannung, Impulsdauer und dergleichen eingebbar sind. Außerdem sind dem programmierbaren Elektromyostimulator 10 die Impulsphasen bzw. Laufzeiten eingebbar. Die Folge von Impulsen 9 gelangt über mehrere Leitungen 11 an mehrere Ausgänge 12, 13 und 14. Je ein Ausgang 12 bis 14 ist für einen Kanal der Stimulationsvorrichtung vorgesehen. Über die Leitungen 15, 16 und 17 gelangen die Impulse zu Elektroden 18 bis 23, die an Manschetten 24 und 25 untergebracht sind. Im einzelnen befinden sich die Elektroden 18 bis 21 an der Manschette 24 und die Elektroden 22 und 23 an der Manschette 25. Außer den Elektroden 18 bis 23 weisen die Manschetten 24, 25 auch je einen nicht dargestellten Klettverschluß auf, mit deren Hilfe sie befestigt werden können. Im Ausführungsbeispiel gemäß der Figur ist die Manschette 24 am Oberschenkel 26 und die Manschette 25 am Unterschenkel 27 des Probanden 5 angebracht.

Die Stimulationsvorrichtung 1 erhält als Input bzw. Eingangssignal ein Herzsignal des Probanden 5. Zwei in der Herzgegend des Probanden 5 befestigte Elektroden 28, 29 greifen dieses Herzsignal ab. Das Herzsignal gelangt über eine Leitung 30 an den Eingangsanschluß 31 der Stimulationsvorrichtung 1. Dieser Eingangsanschluß 31 ist mit dem ersten Modul 2 verbunden, welches aus dem Signal das EKG ableitet.

Ausgehend vom Triggerimpuls 8 erzeugt die Stimulationsvorrichtung 1 unter Berücksichtigung der Laufzeiten eine Folge von Impulsen 9 für die verschiedenen Kanäle, die den Elektroden 18 bis 23 der Manschetten 24, 25 zufließen. Verursacht durch die Impulse kontrahieren die Oberschenkel- bzw. Unterschenkelmuskeln. Dadurch erreicht man nicht nur ein - einer Muskeldegeneration vorbeugendes - Training, sondern es erfolgt auch eine Anregung des Herz-Kreislaufsystems bzw. des Lymphsystems.

Die beschriebene Stimulationsvorrichtung 1 dient einerseits therapeutischen Zwecken. Andererseits ist sie aber auch für diagnostische Zwecke verwendbar. Insbesondere kann man ausgehend von einer vorgegebenen Muskelstimulation die Herzaktivität beobachten. Dabei läßt eine mögliche - durch Muskelstimulation hervorgerufenen - Abweichung des EKG 6 von seinem normalen Signalverlauf Rückschlüsse auf den Gesundheits- oder Trainingszustand des Probanden 5 zu. Die Vorrichtung ist also sowohl für therapeutische als auch für diagnostische Zwecke geeignet.

## Patentansprüche

1. Vorrichtung (1) zum Stimulieren wenigstens eines Muskels oder einer Muskelgruppe für diagnostische und/oder therapeutische Zwecke zur Förderung der Durchblutung in dem stimulierten Muskelbereich (26, 27),
a) welche periodisch elektrische Impulse - wenigstens ein Stimulationssignal (9) - von einem Impulsgenerator (3, 4) über extern auf die Haut des Benutzers an dem zu stimulierenden Bereich (26, 27) plazierten Elektroden (18-23) auf den zu stimulierenden Muskel bzw. die zu stimulierende Muskelgruppe überträgt,
b) wobei die Parameter des Impulsgenerators (3, 4) nämlich Impulsfrequenz, Impulsamplitude, Impulsspannung, Impulsdauer, von mindestens einem Steuergerät (10) aus steuerbar sind,
gekennzeichnet durch
c) eine derartige Auslegung des Steuergerätes (10), daß es zur Steuerung zumindest der Impulsfrequenz ein Steuersignal aus einem bestimmten Bereich periodisch auftretender elektrischer Signalzyklen der Herzaktion(en) - dem Herzsignal (6) - eines Benutzers (5) ableitet,
d) derart, daß zwischen dem Herzsignal (6) und dem Stimulationssignal (9) eine zeitliche Abhängigkeit herrscht, die
e) die Laufzeit einer Blutdruckwelle vom Herzen zum/zur zu stimulierenden Muskel/Muskelgruppe durch entsprechend zeitlich versetzt ausgelöste Impulse (9) berücksichtigt.

2. Vorrichtung (1) nach Anspruch 1, dadurch gekennzeichnet, daß bei der Stimulation von zwei oder mehr Muskel/Muskelgruppen diese mit mehreren, synchronen oder zeitlich versetzten Stimulationssignalen (9), insbesondere mittels eines Multiplexers, stimuliert werden.

3. Vorrichtung (1) nach Anspruch 2, dadurch gekennzeichnet, daß zur Stimulation ein Multiplexer eingesetzt wird.

4. Vorrichtung (1) nach Anspruch 3, dadurch gekennzeichnet, daß die Muskel/Muskelgruppen derart zeitlich versetzt stimuliert werden, daß Blut und/oder Lymphe in eine bestimmte, dem natürlichen Kreislauf entsprechende Richtung transportiert bzw. gepumpt wird.

5. Vorrichtung (1) nach einem der vorstehenden Ansprüche, dadurch gekennzeichnet, daß mehrere Elektrodenpaare (18-23) vorgesehen sind.

6. Vorrichtung (1) nach Anspruch 5, dadurch gekennzeichnet, daß das Steuergerät (10) mehrere Kanäle aufweist, wobei an jeden Kanal ein Elektrodenpaar (18-23) anschließbar ist.

7. Vorrichtung (1) nach einem der vorstehenden Ansprüche, dadurch gekennzeichnet, daß die Elektroden (18-23) zusammen mit wenigstens einer Manschette (24, 25) den zu stimulierenden Bereich (26, 27) umgeben.

8. Vorrichtung (1) nach einem der vorstehenden Ansprüche, dadurch gekennzeichnet, daß das (die) Stimulationssignal(e) (9) abhängig von Gehirnströmen ist (sind).

9. Vorrichtung (1) nach Anspruch 8, dadurch gekennzeichnet, daß Schlafströme als Gehirnströme eingesetzt werden.
